(19) Europäisches Patentamt / European Patent Office / Office européen des brevets

(11) **EP 4 718 459 A1**

(12) **EUROPEAN PATENT APPLICATION**

(43) Date of publication:
01.04.2026 Bulletin 2026/14

(21) Application number: 24465576.7

(22) Date of filing: 25.09.2024

(51) International Patent Classification (IPC):
*G16B 15/30* $^{(2019.01)}$   *G16B 40/20* $^{(2019.01)}$
*G16B 20/20* $^{(2019.01)}$   *G16B 20/30* $^{(2019.01)}$
*G16H 50/20* $^{(2018.01)}$

(52) Cooperative Patent Classification (CPC):
G16B 40/20; G16B 15/30; G16B 20/20;
G16H 50/20

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Siemens Healthineers AG**
**91301 Forchheim (DE)**

(72) Inventors:
• **SIEBERT, Matthias**
**91080 Marloffstein (DE)**
• **PUIU, Andrei**
**507220 Tarlungeni (RO)**
• **SINGH, Vivek**
**Princeton, 08540 (US)**
• **KAMEN, Ali**
**Skillman, 08558 (US)**

(74) Representative: **HKW Intellectual Property PartG mbB**
**Theresienhöhe 12**
**80339 München (DE)**

(54) **PATIENT-SPECIFIC PROTEIN-PROTEIN INTERACTION GRAPH FOR CLINICAL DECISION MAKING**

(57) Systems and methods for performing one or more medical analysis tasks are provided. 1) patient data comprising mutational data of a patient and 2) an initial PPI (protein-protein interaction) graph are received. A patient-specific PPI graph for the patient is generated based on the mutational data and the initial PPI graph. One or more medical analysis tasks for the patient are performed using a machine learning based network based on the patient data and the patient-specific PPI graph. Results of the medical analysis task are output.

**Description**

TECHNICAL FIELD

The present invention relates generally to AI/ML (artificial intelligence/machine learning) based clinical decision making, and in particular to patient-specific protein-protein interaction graph for clinical decision making.

BACKGROUND

The genetic makeup of cells contributes to, or even determines, etiology of many diseases. One example of such disease is cancer, a genetic disease that develops in a multistep process by progressively acquiring somatic mutations in oncogenes and tumor suppressor genes of a tissue that transform a normal cell into a malignant cell. Consequently, genotyping technologies are increasingly being employed to support clinical decision making.

While the genome is the blueprint for cell functioning, it is descriptive in nature. More functional readouts that link genotypes to phenotypes, as provided by 'omics modalities such as transcriptomics and proteomics, are expected to have a greater capacity to inform clinical decision making. However, genomics is the most clinically established 'omics modality.

Recently, predictive models have been proposed for predicting disease etiology based on 'omics data. However, robust training of such predictive models is difficult due to the high-dimensional nature of 'omics data, inherent genetic heterogeneity, and small patient cohorts.

BRIEF SUMMARY OF THE INVENTION

In accordance with one or more embodiments, systems and methods for performing one or more medical analysis tasks are provided. 1) patient data comprising mutational data of a patient and 2) an initial PPI (protein-protein interaction) graph are received. A patient-specific PPI graph for the patient is generated based on the mutational data and the initial PPI graph. One or more medical analysis tasks for the patient are performed using a machine learning based network based on the patient data and the patient-specific PPI graph. Results of the medical analysis task are output. In one embodiment, the initial PPI graph is adjusted based on the mutational data. The initial PPI graph is adjusted by at least one of inserting edges, removing edges, or changing weights of edges of the initial PPI graph based on the mutational data.

In one embodiment, the mutational data comprises data relating to missense mutations of the patient.

In one embodiment, a measure of confidence associated with results of the one or more medical analysis tasks is predicted using the machine learning based network.

In one embodiment, the one or more medical analysis tasks are performed further based on additional data of the patient.

In one embodiment, performing one or more medical analysis tasks for the patient comprises predicting health of the patient.

In one embodiment, the patient data comprises 'omics data of the patient.

In one embodiment, the machine learning based network comprises at least one of a graph neural network or a transformer network.

These and other advantages of the invention will be apparent to those of ordinary skill in the art by reference to the following detailed description and the accompanying drawings.

BRIEF DESCRIPTION OF THE DRAWINGS

Figure 1 shows a method for performing one or more medical analysis tasks based on a patient-specific PPI graph, in accordance with one or more embodiments;

Figure 2 shows a workflow for performing one or more medical analysis tasks based on a patient-specific PPI graph, in accordance with one or more embodiments;

Figure 3 shows an exemplary artificial neural network that may be used to implement one or more embodiments; and

Figure 4 shows a high-level block diagram of a computer that may be used to implement one or more embodiments.

DETAILED DESCRIPTION

The present invention generally relates to methods and systems for patient-specific protein-protein interaction graph based clinical decision making. PPI (protein-protein interaction) refers to the physical contact established between two or more protein molecules. Through PPIs, proteins can perform complex biological functions. Accordingly, understanding PPIs provides valuable insight into clinical decision making for, e.g., diagnosis, prognosis, treatment selection, and monitoring of patients. Recently, it has been found that somatic missense mutations are significantly enriched in PPI interfaces. Furthermore, PPI interface mutations not only disrupt, but also create, new PPIs, termed neoPPIs or oncoPPIs, encompassing both oncogenic and tumor suppressor mutations and, consequently, mediate rewiring of oncogenic programs. Embodiments described herein leverage prior and predicted knowledge of mutations by explicitly modeling PPIs as a graph in a graph neural network and personalizing the graph based on mutations of the patient to provide for a patient-specific PPI graph. Advantageously, such patient-specific PPI graph provides for improved clinical decision making as compared to conventional approaches.

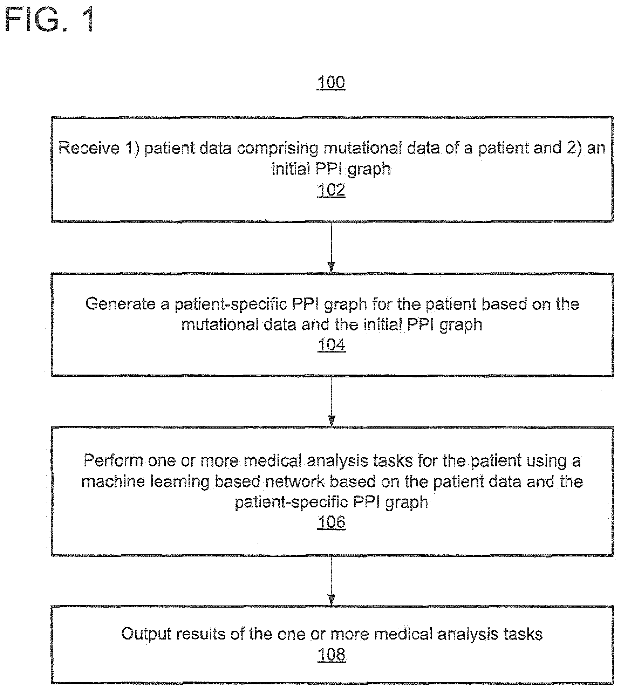

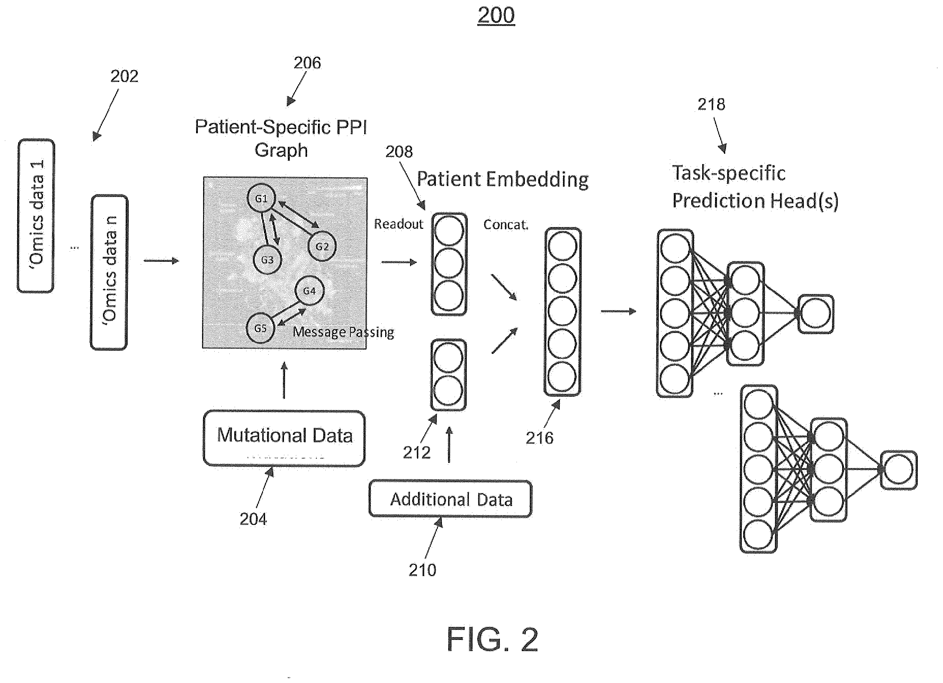

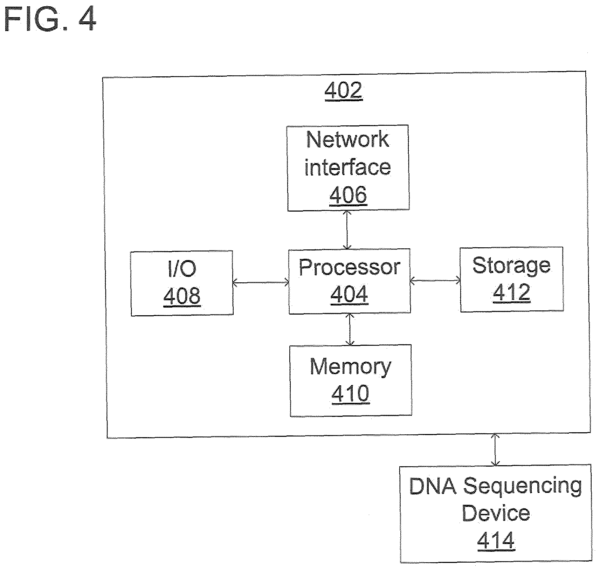

Figure 1 shows a method 100 for performing one or more medical analysis tasks based on a patient-specific PPI graph, in accordance with one or more embodiments. The steps and sub-steps of method 100 may be performed by one or more suitable computing devices, such as, e.g., computer 402 of Figure 4. Figure 2 shows a workflow 200 for performing one or more medical analysis tasks based on a patient-specific PPI graph, in accordance with one or more embodiments. Figure 1 and Figure 2 will be described together.

At step 102 of Figure 1, 1) patient data comprising mutational data of a patient and 2) an initial PPI graph are received. In one example, as shown in workflow 200 of Figure 2, the patient data is 'omics data 202 (comprising 'omics data 1 ... 'omics data n) and mutational data 204 of a patient. It should be understood that mutational data 204 is a type of 'omics data and, in some embodiments, mutational data 204 may be included in 'omics data 202.

The mutational data may comprise data of any patient-specific mutation of the patient known or predicted to affect PPI. One example of mutational data is data relating to patient-specific missense mutations. Missense mutations are a type of genetic mutation where a single nucleotide change in the DNA sequence leads to the substitution of one amino acid for another in the resulting protein. Missense mutations may affect PPIs by altering binding sights affecting its ability to interact with other proteins (loss of interaction or gain of interaction), by changing protein structure and stability, by affecting allosteric sites, or by disrupting protein complexes. Other examples of mutational data include data relating to neoPPIs (neomorph PP!s) or oncoPPIs (oncogenic PPIs). neoPPIs are newly formed PPIs that arise due to mutations that lead to changes in the protein structure. oncoPPIs are PPIs involved in the development, progression, or maintenance of cancer and are typically altered in cancer cells. The mutational data of the patient may be identified using a DNA (deoxyribonucleic acid) sequencing device (e.g., DNA sequencing device 414) by sequencing (subregions of) the patient's genome.

The patient data may comprise any other suitable data of the patient. In one embodiment, the patient data comprises 'omics data. 'omics data refers to any data relating to biological processes and molecules of the patient, such as, for example, data relating to genomics (e.g., data of mutations and copy number variations), transcriptomics, proteomics, metabolomics, epigenomics, lipidomics, microbiomics, or combinations thereof. The 'omics data may be acquired from gene panel sequencing, whole-exome sequencing, RNA (ribonucleic acid) sequencing, methylation sequencing, and/or a mass-spectrometry-based technique.

The initial PPI graph is a generic, non-patient-specific graph modeling PPIs for a patient population. The initial PPI graph comprises nodes and edges connecting the nodes. The nodes represent genes or encoded proteins and the edges represent interactions between the genes or encoded proteins. The genes or proteins may be selected for a disease or medical condition. The edges are weighted to represent a likelihood of a physical association of connected gene or protein pairs. The initial PPI graph may be generated based on a generic, non-patient-specific PPI database (independent of any mutational data). The data of such PPI database may be obtained from experiments measuring physical interactions between proteins. The initial PPI graph may be based on the IAS (integrated association stringency) network or the PIONEER (protein-protein interaction interface prediction) interactome.

The patient data and/or the initial PPI graph may be received, for example, by loading the patient data and/or the initial PPI graph from a storage or memory of a computer system (e.g., storage 412 or memory 410 of computer 402 of Figure 4) or by receiving the patient data and/or the initial PPI graph from a remote computer system (e.g., computer 402 of Figure 4). Such a computer system or remote computer system may comprise one or more patient databases, such as, e.g., an EHR (electronic health record), EMR (electronic medical record), PHR (personal health record), HIS (health information system), RIS (radiology information system), PACS (picture archiving and communication system), LIMS (laboratory information management system), or any other suitable database or system.

At step 104 of Figure 1, a patient-specific PPI graph is generated for the patient based on the mutational data and the initial PPI graph. In one embodiment, the patient-specific PPI graph is generated by adjusting the initial PPI graph based on the mutational data. The initial PPI graph may be adjusted by, for example, inserting or removing edges of the initial PPI graph or by changing weights of edges of the initial PPI graph based on the mutational data.

In one example, as shown in workflow 200 of Figure 2, patient-specific PPI graph 206 is generated based on mutational data 204. Patient-specific PPI graph 206 comprises nodes G1, G2, G3, G4, and G5 and edges connect nodes G1 and G2, G1 and G3, and G4 and G5.

At step 106 of Figure 1, one or more medical analysis tasks are performed for the patient using a machine learning based network based on the patient data and the patient-specific PPI graph.

The machine learning based network may be implemented as using any suitable machine learning based architecture. For example, the machine learning based network may be implemented using a neural network such as, e.g., a GNN (graph neural network) representing PPIs or a transformer architecture that encodes structural information of the PPI graph into a transformer model. The machine learning based network receives as input the patient-specific PPI graph and the patient data and generates as output results of the one or more medical analysis tasks. The machine learning based network is trained during a prior offline or training stage in an end-to-end manner with respect to a diagnostic, prognostic, or predictive conclusion in the context of a disease or medical condition (e.g., cancer). Once trained, the machine learning based network is applied during an online or inference stage, e.g., to perform step 106 of Figure 1.

With reference to workflow 200 of Figure 2, each of the nodes of the patient-specific PPI graph 206 is assigned one or more input features from the patient data (e.g., 'omics data 202 and mutational data 204). Patient-specific PPI graph 206 is implemented with message passing to exchange and aggregate information among nodes as constrained by patient-specific connections, e.g., using graph convolutional networks, graph isomorphism networks, or graph attention networks. Patient-specific PPI graph 206 is then read out into features 208 (e.g., using pooling) by encoding the feature vectors representing the assigned features into features 208. Alternatively, a transformer architecture (e.g., Graphormer) may be used to effectively encode structural information of the patient-specific PPI graph 206 into a transformer model.

In one embodiment, the one or more medical analysis tasks are performed further based on additional patient data 210. Additional patient data 210 may comprise any suitable additional data of the patient. For example, additional patient data 210 may comprise patient information (e.g., age, sex, race), tumor-related data (e.g., location), selected mutations (e.g., KRAS G12V), features (e.g., biomarkers) derived from medical images or laboratory tests of the patient, histologic characteristics, stage of development of a disease, molecular changes detected in tissue or liquid biopsy, etc. As shown in workflow 200, additional patient data 210 is encoded into features 212 using a machine learning based encoder network (e.g., autoencoder). Features 208 and features 212 are then combined (e.g., concatenated) into features 216. Features 208, 212, and 216 are low-level latent features or embeddings representing relationships or structures of the underlying data. It should be understood that the extraction of features 212 and combination of features 208 and 212 is not explicitly performed but is learned end-to-end within the machine learning based network.

Features 216 are fed into task-specific prediction heads 218 to perform the one or more medical analysis tasks. Each task-specific prediction head performs a respective medical analysis task. In one embodiment, the one or more medical analysis tasks comprise a diagnostic, prognostic, or predictive task in the context of a disease or medical condition. For example, the one or more medical analysis task may comprise predicting health of the patient as a value in a clinical decision scale for a disease or medical condition or predicting a next clinical decision for the patient. In another example, the one or more medical analysis task may comprise prediction of local control after radiotherapy treatment of lung cancer patients based on genomics and matched outcome data. However, the one or more medical analysis tasks may comprise any other suitable medical analysis task. In one embodiment, the machine learning based network additionally predicts a measure of confidence associated with results of the one or more medical analysis tasks.

At step 108 of Figure 1, results of the medical analysis task are output. For example, the results of the medical analysis task can be output by displaying the results on a display device of a computer system (e.g., I/O 408 of computer 402 of Figure 4), storing the results on a memory or storage of a computer system (e.g., memory 410 or storage 412 of computer 402 of Figure 4), or by transmitting the results to a remote computer system (e.g., computer 402 of Figure 4).

Embodiments described herein are described with respect to the claimed systems as well as with respect to the claimed methods. Features, advantages or alternative embodiments herein can be assigned to the other claimed objects and vice versa. In other words, claims and embodiments for the systems can be improved with features described or claimed in the context of the respective methods. In this case, the functional features of the method are implemented by physical units of the system.

Furthermore, certain embodiments described herein are described with respect to methods and systems utilizing trained machine learning models, as well as with respect to methods and systems for providing trained machine learning models. Features, advantages or alternative embodiments herein can be assigned to the other claimed objects and vice versa. In other words, claims and embodiments for providing trained machine learning models can be improved with features described or claimed in the context of utilizing trained machine learning models, and vice versa. In particular, datasets used in the methods and systems for utilizing trained machine learning models can have the same properties and features as the corresponding datasets used in the methods and systems for providing trained machine learning models, and the trained machine learning models provided by the respective methods and systems can be used in the methods and systems for utilizing the trained machine learning models.

In general, a trained machine learning model mimics cognitive functions that humans associate with other human minds. In particular, by training based on training data the machine learning model is able to adapt to new circumstances and to detect and extrapolate patterns. Another term for "trained machine learning model" is "trained function."

In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used.

In particular, a machine learning model, such as, e.g., the machine learning based model utilized at step 106 of Figure 1 and in workflow 200 of Figure 2, can comprise, for example, a neural network, a support vector machine, a decision tree and/or a Bayesian network, and/or the machine learning model can be based on, for example, k-means clustering, Q-learning, genetic algorithms and/or association rules. In particular, a neural network can be, e.g., a deep neural network, a convolutional neural network or a convolutional deep neural network. Furthermore, a neural network can be, e.g., an adversarial network, a deep adversarial network and/or a generative adversarial network.

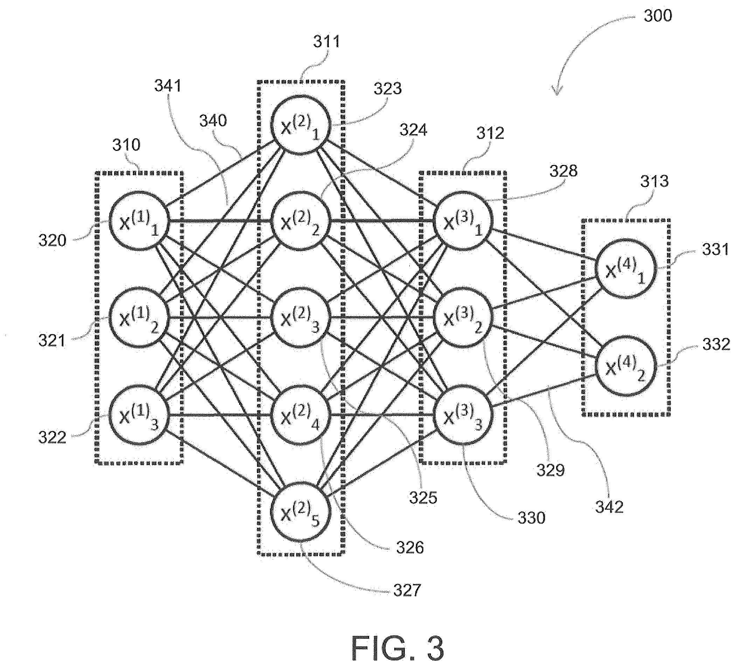

Figure 3 shows an embodiment of an artificial neural network 300 that may be used to implement one or more machine learning models described herein. Alternative terms for "artificial neural network" are "neural network", "artificial neural net" or "neural net".

The artificial neural network 300 comprises nodes 320, ..., 332 and edges 340, ..., 342, wherein each edge 340, ..., 342 is a directed connection from a first node 320, ..., 332 to a second node 320, ..., 332. In general, the first node 320, ..., 332 and the second node 320, ..., 332 are different nodes 320, ..., 332, it is also possible that the first node 320, ..., 332 and the second node 320, ..., 332 are identical. For example, in Figure 3 the edge 340 is a directed connection from the node 320 to the node 323, and the edge 342 is a directed connection from the node 330 to the node 332. An edge 340, ..., 342 from a first node 320, ..., 332 to a second node 320, ..., 332 is also denoted as "ingoing edge" for the second node 320, ..., 332 and as "outgoing edge" for the first node 320, ..., 332.

In this embodiment, the nodes 320, ..., 332 of the artificial neural network 300 can be arranged in layers 310, 313, wherein the layers can comprise an intrinsic order introduced by the edges 340, ..., 342 between the nodes 320, ..., 332. In particular, edges 340, ..., 342 can exist only between neighboring layers of nodes. In the displayed embodiment, there is an input layer 310 comprising only nodes 320, ..., 322 without an incoming edge, an output layer 313 comprising only nodes 331, 332 without outgoing edges, and hidden layers 311, 312 in-between the input layer 310 and the output layer 313. In general, the number of hidden layers 311, 312 can be chosen arbitrarily. The number of nodes 320, ..., 322 within the input layer 310 usually relates to the number of input values of the neural network, and the number of nodes 331, 332 within the output layer 313 usually relates to the number of output values of the neural network.

In particular, a (real) number can be assigned as a value to every node 320, ..., 332 of the neural network 300. Here, $x^{(n)}_i$ denotes the value of the i-th node 320, ..., 332 of the n-th layer 310, ..., 313. The values of the nodes 320, ..., 322 of the input layer 310 are equivalent to the input values of the neural network 300, the values of the nodes 331, 332 of the output layer 313 are equivalent to the output value of the neural network 300. Furthermore, each edge 340, ..., 342 can comprise a weight being a real number, in particular, the weight is a real number within the interval [-1, 1] or within the interval [0, 1]. Here, $w^{(m,n)}_{i,j}$ denotes the weight of the edge between the i-th node 320, ..., 332 of the m-th layer 310, ..., 313 and the j-th node 320, ..., 332 of the n-th layer 310, ..., 313. Furthermore, the abbreviation $w^{(n)}_{i,j}$ is defined for the weight $w^{(n,n+1)}_{i,j}$.

In particular, to calculate the output values of the neural network 300, the input values are propagated through the neural network. In particular, the values of the nodes 320, ..., 332 of the (n+1)-th layer 310, ..., 313 can be calculated based on the values of the nodes 320, ..., 332 of the n-th layer 310, ..., 313 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 310 are given by the input of the neural network 300, wherein values of the first hid-den layer 311 can be calculated based on the values of the input layer 310 of the neural network, wherein values of the second hidden layer 312 can be calculated based in the values of the first hidden layer 311, etc.

In order to set the values $w^{(m,n)i,j}$ for the edges, the neural network 300 has to be trained using training data. In particular, training data comprises training input data and training output data (denoted as ti). For a training step, the neural network 300 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 300 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta^{(n)_j} = \left( \sum_k \delta^{(n+1)_k} \cdot w^{(n+1)_{j,k}} \right) \cdot f'\left( \sum_i x^{(n)_i} \cdot w^{(n)_{i,j}} \right)$$

based on $\delta^{(n+1)}_j$, if the (n+1)-th layer is not the output layer, and

$$\delta^{(n)_j} = \left( x^{(n+1)_j} - t^{(n+1)_j} \right) \cdot f'\left( x^{(n)_i} \cdot w^{(n)_{i,j}} \right)$$

if the (n+1)-th layer is the output layer 313, wherein f' is the first derivative of the activation function, and $t^{(n+1)}_j$ is the comparison training value for the j-th node of the output layer 313.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatuses, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatuses, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1 or 2. Certain steps or functions of the methods and workflows described herein, including one or more of the steps or functions of Figures 1 or 2, may be performed by a server or by another processor in a network-based cloud-computing system. Certain steps or functions of the methods and workflows described herein, including one or more of the steps of Figures 1 or 2, may be performed by a client computer in a network-based cloud computing system. The steps or functions of the methods and workflows described herein, including one or more of the steps of Figures 1 or 2, may be performed by a server and/or by a client computer in a network-based cloud computing system, in any combination.

Systems, apparatuses, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1 or 2, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 402 that may be used to implement systems, apparatuses, and methods described herein is depicted in Figure 4. Computer 402 includes a processor 404 operatively coupled to a data storage device 412 and a memory 410. Processor 404 controls the overall operation of computer 402 by executing computer program instructions that define such operations. The computer program instructions may be stored in data storage device 412, or other computer readable medium, and loaded into memory 410 when execution of the computer program instructions is desired. Thus, the method and workflow steps or functions of Figures 1 or 2 can be defined by the computer program instructions stored in memory 410 and/or data storage device 412 and controlled by processor 404 executing the computer program instructions. For example, the computer program instructions can be implemented as computer executable code programmed by one skilled in the art to perform the method and workflow steps or functions of Figures 1 or 2. Accordingly, by executing the computer program instructions, the processor 404 executes the method and workflow steps or functions of Figures 1 or 2. Computer 402 may also include one or more network interfaces 406 for communicating with other devices via a network. Computer 402 may also include one or more input/output devices 408 that enable user interaction with computer 402 (e.g., display, keyboard, mouse, speakers, buttons, etc.).

Processor 404 may include both general and special purpose microprocessors, and may be the sole processor or one of multiple processors of computer 402. Processor 404 may include one or more central processing units (CPUs), for example. Processor 404, data storage device 412, and/or memory 410 may include, be supplemented by, or incorporated in, one or more application-specific integrated circuits (ASICs) and/or one or more field programmable gate arrays (FPGAs).

Data storage device 412 and memory 410 each include a tangible non-transitory computer readable storage medium. Data storage device 412, and memory 410, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 408 may include peripherals, such as a printer, scanner, display screen, etc. For example, input/output devices 408 may include a display device such as a cathode ray tube (CRT) or liquid crystal display (LCD) monitor for displaying information to the user, a keyboard, and a pointing device such as a mouse or a trackball by which the user can provide input to computer 402.

A DNA sequencing device 414 can be connected to the computer 402 to determine the sequence of nucleotides (Adenine, Thymine, Cytosine, and Guanine) in a DNA molecule. DNA sequencing may be used for, e.g., sequencing (subregions of) a patient's genome to identify patient-specific mutational data or providing additional 'omics data, which may be input to the computer 402.

Any or all of the systems, apparatuses, and methods discussed herein may be implemented using one or more computers such as computer 402.

One skilled in the art will recognize that an implementation of an actual computer or computer system may have other structures and may contain other components as well, and that Figure 4 is a high level representation of some of the components of such a computer for illustrative purposes.

Independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope and spirit of the invention. Those skilled in the art could implement various other feature combinations without departing from the scope and spirit of the invention.

The following is a list of non-limiting illustrative embodiments disclosed herein:

Illustrative embodiment 1. A computer-implemented method comprising: receiving 1) patient data comprising mutational data of a patient and 2) an initial PPI (protein-protein interaction) graph; generating a patient-specific PPI graph for the patient based on the mutational data and the initial PPI graph; performing one or more medical analysis tasks for the patient using a machine learning based network based on the patient data and the patient-specific PPI graph; and outputting results of the medical analysis task.

Illustrative embodiment 2. The computer-implemented method of illustrative embodiment 1, wherein generating a patient-specific PPI graph for the patient based on the mutational data and the initial PPI graph comprises: adjusting the initial PPI graph based on the mutational data.

Illustrative embodiment 3. The computer-implemented method of illustrative embodiment 2, wherein adjusting the initial PPI graph based on the mutational data comprises at least one of inserting edges, removing edges, or changing weights of edges of the initial PPI graph based on the mutational data.

Illustrative embodiment 4. The computer-implemented method of any of illustrative embodiments 1-3, wherein the mutational data comprises data relating to missense mutations of the patient.

Illustrative embodiment 5. The computer-implemented method of any of illustrative embodiments 1-4, further comprising: predicting a measure of confidence associated with results of the one or more medical analysis tasks using the machine learning based network.

Illustrative embodiment 6. The computer-implemented method of any of illustrative embodiments 1-5, wherein performing one or more medical analysis tasks for the patient using a machine learning based network based on the patient data and the patient-specific PPI graph comprises: performing the one or more medical analysis tasks further based on additional data of the patient.

Illustrative embodiment 7. The computer-implemented method of any of illustrative embodiments 1-6, wherein performing one or more medical analysis tasks for the patient using a machine learning based network based on the patient data and the patient-specific PPI graph comprises: predicting health of the patient.

Illustrative embodiment 8. The computer-implemented method of any of illustrative embodiments 1-7, wherein the patient data comprises 'omics data of the patient.

Illustrative embodiment 9. The computer-implemented method of any of illustrative embodiments 1-8, wherein the machine learning based network comprises at least one of a graph neural network or a transformer network.

Illustrative embodiment 10. An apparatus comprising: means for receiving 1) patient data comprising mutational data of a patient and 2) an initial PPI (protein-protein interaction) graph; means for generating a patient-specific PPI graph for the patient based on the mutational data and the initial PPI graph; means for performing one or more medical analysis tasks for the patient using a machine learning based network based on the patient data and the patient-specific PPI graph; and means for outputting results of the medical analysis task.

Illustrative embodiment 11. The apparatus of illustrative embodiment 10, wherein the means for generating a patient-specific PPI graph for the patient based on the mutational data and the initial PPI graph comprises: means for adjusting the initial PPI graph based on the mutational data.

Illustrative embodiment 12. The apparatus of illustrative embodiment 11, wherein the means for adjusting the initial PPI graph based on the mutational data comprises at least one of means for inserting edges, means for removing edges, or means for changing weights of edges of the initial PPI graph based on the mutational data.

Illustrative embodiment 13. The apparatus of any of illustrative embodiments 10-12, wherein the mutational data comprises data relating to missense mutations of the patient.

Illustrative embodiment 14. The apparatus of any of illustrative embodiments 10-13, further comprising: means for predicting a measure of confidence associated with results of the one or more medical analysis tasks using the machine learning based network.

Illustrative embodiment 15. A non-transitory computer-readable storage medium comprising instructions which, when executed by a computer, cause the computer to carry out operations comprising: receiving 1) patient data comprising mutational data of a patient and 2) an initial PPI (protein-protein interaction) graph; generating a patient-specific PPI graph for the patient based on the mutational data and the initial PPI graph; performing one or more medical analysis tasks for the patient using a machine learning based network based on the patient data and the patient-specific PPI graph; and outputting results of the medical analysis task.

Illustrative embodiment 16. The non-transitory computer-readable storage medium of illustrative embodiment 15, wherein generating a patient-specific PPI graph for the patient based on the mutational data and the initial PPI graph comprises: adjusting the initial PPI graph based on the mutational data.

Illustrative embodiment 17. The non-transitory computer-readable storage medium of any of illustrative embodiments 15-16, wherein performing one or more medical analysis tasks for the patient using a machine learning based network based on the patient data and the patient-specific PPI graph comprises: performing the one or more medical analysis tasks further based on additional data of the patient.

Illustrative embodiment 18. The non-transitory computer-readable storage medium of any of illustrative embodiments 15-17, wherein performing one or more medical analysis tasks for the patient using a machine learning based network based on the patient data and the patient-specific PPI graph comprises: predicting health of the patient.

Illustrative embodiment 19. The non-transitory computer-readable storage medium of any of illustrative embodiments 15-18, wherein the patient data comprises 'omics data of the patient.

Illustrative embodiment 20. The non-transitory computer-readable storage medium of any of illustrative embodiments 15-19, wherein the machine learning based network comprises at least one of a graph neural network or a transformer network.

**Claims**

1. A computer-implemented method comprising:

    receiving (102) 1) patient data comprising mutational data (204) of a patient and 2) an initial PPI (protein-protein interaction) graph;
    generating (104) a patient-specific PPI graph (206) for the patient based on the mutational data and the initial PPI graph;

performing (106) one or more medical analysis tasks for the patient using a machine learning based network based on the patient data and the patient-specific PPI graph; and

outputting (108) results of the medical analysis task.

2. The computer-implemented method of claim 1, wherein generating a patient-specific PPI graph for the patient based on the mutational data and the initial PPI graph comprises:
adjusting the initial PPI graph based on the mutational data.

3. The computer-implemented method of claim 2, wherein adjusting the initial PPI graph based on the mutational data comprises at least one of inserting edges, removing edges, or changing weights of edges of the initial PPI graph based on the mutational data.

4. The computer-implemented method of claim 1, wherein the mutational data comprises data relating to missense mutations of the patient.

5. The computer-implemented method of claim 1, further comprising:
predicting a measure of confidence associated with results of the one or more medical analysis tasks using the machine learning based network.

6. The computer-implemented method of claim 1, wherein performing one or more medical analysis tasks for the patient using a machine learning based network based on the patient data and the patient-specific PPI graph comprises:
performing the one or more medical analysis tasks further based on additional data (210) of the patient

7. The computer-implemented method of claim 1, wherein performing one or more medical analysis tasks for the patient using a machine learning based network based on the patient data and the patient-specific PPI graph comprises:
predicting health of the patient.

8. The computer-implemented method of claim 1, wherein the patient data comprises 'omics data (202) of the patient.

9. The computer-implemented method of claim 1, wherein the machine learning based network comprises at least one of a graph neural network or a transformer network.

10. An apparatus comprising:

means for receiving (102) 1) patient data comprising mutational data (204) of a patient and 2) an initial PPI (protein-protein interaction) graph;
means for generating (104) a patient-specific PPI graph (206) for the patient based on the mutational data and the initial PPI graph;
means for performing (106) one or more medical analysis tasks for the patient using a machine learning based network based on the patient data and the patient-specific PPI graph; and
means for outputting (108) results of the medical analysis task.

11. The apparatus of claim 10, wherein the means for generating a patient-specific PPI graph for the patient based on the mutational data and the initial PPI graph comprises:
means for adjusting the initial PPI graph based on the mutational data.

12. The apparatus of claim 11, wherein the means for adjusting the initial PPI graph based on the mutational data comprises at least one of means for inserting edges, means for removing edges, or means for changing weights of edges of the initial PPI graph based on the mutational data.

13. The apparatus of claim 10, wherein the mutational data comprises data relating to missense mutations of the patient.

14. The apparatus of claim 10, further comprising:
means for predicting a measure of confidence associated with results of the one or more medical analysis tasks using the machine learning based network.

15. A non-transitory computer-readable storage medium comprising instructions which, when executed by a computer, cause the computer to carry out operations comprising:

receiving (102) 1) patient data comprising mutational data (204) of a patient and 2) an initial PPI (protein-protein interaction) graph;

generating (104) a patient-specific PPI graph (206) for the patient based on the mutational data and the initial PPI graph;

performing (106) one or more medical analysis tasks for the patient using a machine learning based network based on the patient data and the patient-specific PPI graph; and

outputting (108) results of the medical analysis task.

16. The non-transitory computer-readable storage medium of claim 15, wherein generating a patient-specific PPI graph for the patient based on the mutational data and the initial PPI graph comprises:
adjusting the initial PPI graph based on the mutational data.

17. The non-transitory computer-readable storage medium of claim 15, wherein performing one or more medical analysis tasks for the patient using a machine learning based network based on the patient data and the patient-specific PPI graph comprises:
performing the one or more medical analysis tasks further based on additional data (210) of the patient.

18. The non-transitory computer-readable storage medium of claim 15, wherein performing one or more medical analysis tasks for the patient using a machine learning based network based on the patient data and the patient-specific PPI graph comprises:
predicting health of the patient.

19. The non-transitory computer-readable storage medium of claim 15, wherein the patient data comprises 'omics data (202) of the patient.

20. The non-transitory computer-readable storage medium of claim 15, wherein the machine learning based network comprises at least one of a graph neural network or a transformer network.

# FIG. 1

100

Receive 1) patient data comprising mutational data of a patient and 2) an
initial PPI graph
102

Generate a patient-specific PPI graph for the patient based on the
mutational data and the initial PPI graph
104

Perform one or more medical analysis tasks for the patient using a
machine learning based network based on the patient data and the
patient-specific PPI graph
106

Output results of the one or more medical analysis tasks
108

FIG. 2

FIG. 3

FIG. 4

```
                            402

              ┌─────────────────┐
              │     Network     │
              │    interface    │
              │       406       │
              └─────────────────┘
                       ↕
┌──────────┐    ┌─────────────┐    ┌──────────┐
│   I/O    │↔   │  Processor  │  ↔ │ Storage  │
│   408    │    │     404     │    │   412    │
└──────────┘    └─────────────┘    └──────────┘
                       ↕
              ┌─────────────────┐
              │     Memory      │
              │       410       │
              └─────────────────┘
```

┌────────────────────┐
│  DNA Sequencing    │
│      Device        │
│       414          │
└────────────────────┘

# EUROPEAN SEARCH REPORT

Europäisches
Patentamt
European
Patent Office
Office européen
des brevets

**Application Number**

EP 24 46 5576

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZITNIK MARINKA ET AL: "Current and future directions in network biology", BIOINFORMATICS ADVANCES, vol. 4, no. 1, 14 August 2024 (2024-08-14), XP093254194, ISSN: 2635-0041, DOI: 10.1093/bioadv/vbae099 | 1-4, 6-13, 15-20 | INV. G16B15/30 G16B40/20 G16B20/20 G16B20/30 G16H50/20 |
| Y | * whole document, in particular Figures; paragraph "Inference of a condition-specific network"; paragraph "5. Machine learning on networks"; paragraph "6. Network-based personalized medicine" * ----- | 5,14 | |
| X | QIU JIAJUN ET AL: "Network-based protein-protein interaction prediction method maps perturbations of cancer interactome", PLOS GENETICS, vol. 17, no. 11, 2 November 2021 (2021-11-02), page e1009869, XP093253996, US ISSN: 1553-7404, DOI: 10.1371/journal.pgen.1009869 | 1-20 | |
| Y | * whole document, in particular authors summary; Figures; Materials and methods * ----- | 5,14 | |
| A | ZHOU YUNZHUO ET AL: "DDMut-PPI: predicting effects of mutations on protein-protein interactions using graph-based deep learning", NUCLEIC ACIDS RESEARCH, vol. 52, no. W1, 23 May 2024 (2024-05-23), pages W207-W214, XP093253352, GB ISSN: 0305-1048, DOI: 10.1093/nar/gkae412 * whole document, in particular abstract; Materials and methods; Validation; Figures * ----- | 1-20 | |

**TECHNICAL FIELDS SEARCHED (IPC)**

G16B
G16H

The present search report has been drawn up for all claims

| Place of search | Date of completion of the search | Examiner |
|---|---|---|
| Munich | 26 February 2025 | Vanmontfort, D |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................
& : member of the same patent family, corresponding
document

EPO FORM 1503 03.82 (P4C01)